# EUROPEAN PATENT APPLICATION

(11) **EP 2 889 364 A1**
(43) Date of publication of application: **01.07.2015**
(21) Application number: 14460132.5
(22) Date of filing: 23.12.2014
(51) Int. Cl.: C12M 1/107, C12M 1/12, C12M 1/26, C12M 1/34

(54) **METHOD AND SYSTEM FOR METHANE PRODUCTION AND DISPOSAL OF CARBON DIOXIDE**

(30) Priority: 24.12.2013 PL 40670313; 24.12.2013 PL 40670413
(71) Applicant: SYGMA Sp. z o.o., 54-211 Wroclaw (PL); Lukaszewicz, Marcin Ziemowit, 51-351 Wroclaw (PL); Sygit, Maciej, 54-211 Wroclaw (PL)
(72) Inventor: Lukaszewicz, Marcin Ziemowit, 51-351 Wroc?aw (PL); Sygit, Maciej, 54-211 Wroc?aw (PL)
(74) Representative: Winohradnik, J. Halina

(57) **Abstract**

The subject matter of the invention is a method and a system of methane production and disposal of waste carbon dioxide, comprising a methane digestion of organic matter, and consisting in that the organic matter microbiological decomposition proceeds under the pressure that is increasing during the process run in a tubular bioreactor divided into spontaneously separating zones. In the first zone (4), of increasing hydrostatic pressure, the anaerobic digestion of organic matter occurs, and the produced methane is periodically removed to the methane tank (7). Sedimentation and separation of the fraction requiring longer digestion time takes place in the lower bioreactor zone, which fraction is subject to the digestion for an extended time in the zone (8) - the lowest located zone in the bioreactor-while the mineral and non-biodegradable constituents are periodically removed. In the last bioreactor zone (5), where the hydrostatic pressure is decreasing, the evolving gaseous carbon dioxide is removed to a gas tank and the digestate is separated. Furthermore, the carbon dioxide removed from the system together with the digestate and non-biodegradable waste, is stored under pressure underground, in rock mass voids or within a porous rock or at the bottom of a water reservoir.

## Description

The subject matter of the invention is a method and system for methane production by organic matter anaerobic digestion process and disposal of waste carbon dioxide.

Various solutions regarding anaerobic digestion of organic matter such as wastes and by-products from different sectors and branches of production have been known and used so far, with the purpose to produce a biogas containing 50% to 70% of a valuable energy carrier which is methane (CH₄).

The biogas is produced most often in large-size digesters where a substrate is subject to microorganisms action, which process takes many days for the biogas to be produced. In use are also systems, wherein the readily- and hardly-decomposing substrates are subject to pre-processing before they get into a biogas reactor and undergo a microbiological decomposition. Some existing biogasworks are provided with digesters supplied with all substrates, wherefrom the substrates are fed into secondary digesters, During the anaerobic decomposition of organic matter, the hydrogen contained in the organic matter is released in form of methane and the oxygen - in form of carbon dioxide.

A method and a devise for mixing liquid biomass in a bioreactor, especially in the presence of mesophylic and/or thermophylic microorganisms while producing gaseous methane, the device in particular for mixing sewage sludge in separate sealed digestion chambers, have been known from the Polish patent specification No. PL 176200. The method consists in that the mixing proceeds in two consecutive phases: in the ascending phase - in which the biomass is fed from below into an upper tank through an ascending pipe as a result of vacuum produced in this upper tank and where the biomass piles up, and in the recirculation phase - when at least most part of the biomass previously piled-up in the upper tank, under its own pressure in the upper tank, passes into upper layers of the biomass accumulated in the reactor, whereas duration of the recirculation phase is shorter then the ascending phase. Gas pumping out from the upper tank starts at the moment or after the biomass has reached a lower filling level in the upper tank, thus generating a negative pressure in the upper tank, and the biomass from the bioreactor is being drawn therein, so the biomass level in the upper tank rises. In the device for implementing this method the upper end of the ascending pipe is placed in the upper tank, wherefrom at least one circulation pipe reversibly blocked is led out for carrying away the biomass from the upper tank back to the upper part of the bioreactor. The upper tank is located inside and/or above the bioreactor and is provided with a gas inlet pipe and a gas outlet pipe fitted with a vacuum pump. The outlet pipe fitted with a vacuum pump is connected to the upper part of the bioreactor.

Device known from the American patent specification No. US 4735724 for anaerobic digestion of organic matter, provided is with an upright digestion tower, where three zones are spontaneously created through an inertial concentration of biodegradable solid matter and microorganisms. A zone of formation of acids and beneath situated zone of methane formation are located in the upper part of the tower, while its middle and lower part constitute a zone of low concentration of solid constituents. The feed enters from the top, and a part of digested biomass of low solids content, that is leaving the tower, is recirculated to the upper part of the upright tower, where it is evenly sprayed over the top biomass layer. This way the retention time for bacteria and solids from the feed relative to the hydraulic retention time becomes longer, which leads to more complete biodegradation of the feed constituents and to improvement of the bioconversion rate and efficiency. In practice, the correct operation of such upright tower is in jeopardy because of its instability and unpredictability, especially with regard for build-up of a drying up scum in the upper part of the tower, resulting in disturbance of the whole digestion process.

Depending on elemental composition of the organic matter, its oxidation and hydrogenation state, around 30% to 50% of organic carbon is converted to carbon dioxide, the main greenhouse effect offender. Methods of carbon dioxide sequestration or its conversion and storage are known, among others by using the nuclear power. Known is the method of stripping the carbon dioxide, contained in the biogas, from the methane gas, based on significant difference in solubility of both gases in water environment, the method wherein the biogas is passed through pressure tanks filled with water, and the carbon dioxide dissolves under pressure of 30 atm. Then the carbonized water is subject to degasing by reducing pressure, thus releasing the dissolved carbon dioxide to the air.

The essence of the method consists in that the methane digestion of organic matter is carried out while increasing the pressure during the microbiological decomposition in a tubular bioreactor divided into spontaneously separated zones. The anaerobic decomposition of organic matter occurs in the zone of increasing hydrostatic pressure, sedimentation follows in the zone below, in the lowest zone decomposition of the slow-decomposing fraction continues, while mineral and non-biodegradable constituents are removed periodically, and from the last zone, where the hydrostatic pressure is being reduced, the evolving gaseous carbon dioxide is removed to a gas tank and the digestate is separated. According to the method, methane released as a result of anaerobic digestion of organic compounds and accumulating in the bioreactor above the methanisation zone, is periodically carried away to the methane tank, whereas in the sedimentation zone the fraction requiring longer biodegradation time is separated from the quick-decomposing fraction and is subject to digestion in the lowest situated bioreactor zone. Pressure changes during the microbiological decomposition as a result of growing amount of methane and CO₂ in different parts of the bioreactor produce pulsating reciprocating flow of the liquid fraction between reaction zones, which promotes mixing the reagents with microorganisms. CO₂ evolving from the reaction mixture is received into the carbon dioxide tank, while the separated digestate containing carbon dioxide (CO₂) dissolved therein, flows to the digestate tank. The substrate is fed to the bioreactor, and digestate removed therefrom in accordance with the substrate conversion rate.

In one variant of the method the process proceeds in an underground excavation, and the produced carbon dioxide together with the digestate and non-biodegradable waste are received from the bottom part of the bioreactor. According to the method, the produced digestate together with dissolved carbon dioxide are carried away outside the system, or stored under pressure underground in rock mass voids or within a porous rock or at the bottom of a considerably deep water reservoir.

In the other variant of the method, the process proceeds in a plant located at a mountain-slope or mounted on a structure that is high enough to provide a big difference of levels between the sedimentation zone and the methanisation zone. Furthermore, all produced carbon dioxide is removed together with the digestate and non-biodegradable waste to the digestate tank where the pressure is much lower, or is stored in the rocks.

The system for the biogas production comprises a bioreactor in form of a long pipe or pipes connected in shape of a letter "U", where spontaneously separating zones are formed as the organic matter processing occurs. The bioreactor in its upper part is connected to a substrate tank, through a substrate metering pump and a substrate check valve. The upper part of the bioreactor, including the organic matter anaerobic digestion zone, is connected to a biogas tank, through a gas valve, for receiving the obtained methane, separated from a liquid phase, whereas the lower part of the bioreactor, including a sedimentation zone, ends in a chamber for a slow-decomposing fraction, and in a further bottom part of the bioreactor, in the zone where the hydrostatic pressure reduces as the process proceeds, the CO₂ is released and the digestate separated. The sedimentation zone, integrated with the digestion chamber for slow-decomposing fraction, is provided with a set of pipelines and valves to control the removal of mineral and non-biodegradable constituents and CO₂ from the reaction environment.

Preferably, in the bioreactor shaped as a "U"-pipe, the first arm, including the organic matter anaerobic digestion zone, is connected at the top to the methane tank, while the second arm, with the hydrostatic pressure reduction zone, is connected at the top to the tank for storage of CO₂ containing traces of methane, and additionally is provided with an outflow pipe, removing the CO₂-saturated digestate to a receiver. As a result of pressure changes in the bioreactor, mixing the reagents in the system is achieved through a reciprocating movement of the reaction mixture between the bioreactor arms.

The gas valves in the system, preferably computer-controlled, render it possible to maintain optimal pressure during the processing and have an effect on reciprocating movement of the reaction mixture in the bioreactor. The digestate valves provided in the system make possible to periodically carry away the digestate as well as mineral and non-biodegradable constituents.

In a variant of the solution, the receiver is in form of a digestate tank, connected to a chamber of the slow-decomposing sedimenting fraction, preferably through a non-return valve.

The possible digestate receivers may include: a waste tank or a porous rock or an underground excavation or water reservoirs, wherein the accumulated carbon dioxide saturated digestate and the non-biodegradable wastes remain under pressure.

The method and the system according to the invention provide the carbon dioxide sequestration using a biological method, by application of the methane digestion, and the limitation of the greenhouse effect without further energy input makes the additional advantage. The solution provides a very good natural method of oxidised carbon storage, e.g. in coal beds, wherefrom it was previously mined as a fossil fuel. Moreover, the carbon dioxide produced in the methane digestion process is immobilised in those geological beds, which contain sufficient amounts of metal ions, such as calcium or magnesium ions, that enter in reaction with the carbonic acid, formed of a dissolved carbon dioxide. Salts such as calcium carbonate and magnesium carbonate, being slightly soluble in water solutions, are suitable for industrial applications.

The advantage of the plant according to the invention consists in creation of conditions for running the biodegradation process in a pulsating manner while at the same time dividing the processed substrate into a quick-decomposing fraction and the fraction requiring longer digestion time.

The essential feature of the system according to the invention is a great difference in the height level between the inlet and the lowest situated part of the bioreactor, owing to which as the pressure in the lower part of the bioreactor increases, the methanogenesis proceeds faster and more effectively, and so the methane content in the biogas increases, whereas the carbon dioxide content becomes reduced.

The important and desired feature of the system, besides its simple design and low operating costs, is elimination of agitators and possibility to adjust pressure in the system through the application of a computer control function for controlling operation of the valves.

The biogas produced by the method according to the invention is richer in methane content relative to the biogas produced in systems where the digestion process is run under near-atmospheric pressure.

The system according to the invention is designed for its placement underground, in a pit-shaft or geological well, at a mountain slope or under water, and also on high supporting structures.

The object of the invention has been explained in embodiments and in the drawings, where the Fig. 1 presents a system wherein the bioreactor pipes are connected in shape of a letter "U", Fig. 2 - a system wherein the bioreactor is shaped in form of a letter "U", and is provided with one biogas tank and digestate receiver, Fig. 3 - a system provided with a bioreactor in form of a long pipe and a rock receiver, and Fig. 4 - a system provided with a bioreactor in form of a long pipe and with a waste tank.

### Embodiment 1

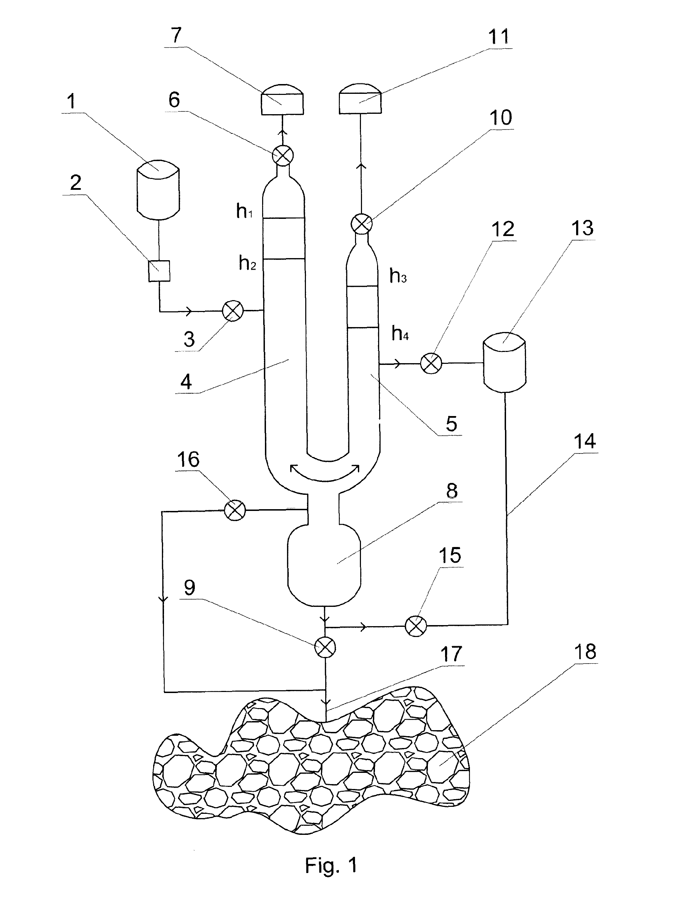

The system (visualised in Fig. 1) comprises a substrate tank **1,** from which through a metering pump **2** and a non-return valve **3**, the substrate is delivered to the organic matter anaerobic digestion zone, located in the first arm **4** of the bioreactor, shaped in form of a letter "U", whereas in the second arm **5** thereof, of a smaller diameter and height, constituting communicating vessels with the first one, a zone of decreasing hydrostatic pressure is situated. The substrate feed pipe is located in the upper part of the first bioreactor arm **4,** whereas a computer-controlled gas valve **6** and a biogas tank 7 serve for receiving the biogas, mostly methane containing, produced in a methanisation zone. A lower part of the bioreactor "U-pipe", wherein located is a sedimentation zone, is integrated with a chamber **8,** connecting both arms and containing a slow-decomposing sedimenting fraction, digestion of which fraction continues therein, while the mineral and non-biodegradable constituents are periodically removed therefrom by a pipeline provided with a computer-controlled valve **9.** In a decreasing hydrostatic pressure zone, located in the second arm **5,** the carbon dioxide containing traces of methane and produced in the anaerobic digestion process, is removed through a gas valve **10** and received to a biogas tank **11.** In the second arm **5,** a digestate outlet is located slightly below the substrate inlet level into the bioreactor, and connected through the digestate valve **12** to the digestate tank **13.** Alternatively, to the same tank, by a discharge pipeline **14,** and through the valve **15,** removed are mineral and non-biodegradable constituents from the chamber **8** for the slow-decomposing sedimenting fraction. Furthermore, a part of separated non-biodegradable solid constituents is periodically removed from the sedimentation zone, while skipping the chamber **8,** and is delivered through the valve **16** and the pipeline **17** to the receiver **18,** which is a porous rock, accumulating under pressure the waste saturated with carbon dioxide.

The microbiological decomposition with participation of microorganisms, in spontaneously forming reaction zones proceeds under increasing pressure in a system, wherein the substrate from the tank **1** is metered to the methanogenesis zone situated in the first bioreactor arm **4,** through a pump **2,** while the valves **6, 9, 10, 12, 15** and **16** remain closed, and the valve **3** is open, until the substrate table level reaches the level **h**₁. If at the same time the level **h₃** is not reached, opens the valve **10** while the pump **2** remains turned on.

The pump **2** and the valve **10** to be switched off and the substrate metering to the reactor stops when the substrate table level reaches the value **h₃.** As the microbiological decomposition of organic matter in both bioreactor arms **4** and **5** progresses, the liquid phase table levels change, in the first bioreactor arm **4** - between **h₁** and **h₂** and in the second bioreactor arm **5** between **h₄** and **h₃**. The methane produced as a result of the anaerobic digestion is separated in the upper part of the bioreactor arm **4,** and when the level in the upper part of the arm **4** drops at least to **h₂** as a result of rising gas pressure, the gas valve **6** opens, releasing the methane flow to the gas tank **7.** Operation of the valves **6** and **10** is computer-controlled in such a manner that the reference table levels of the liquid phase are changing alternately, and when the substrate table level in the chamber **4** reaches the level **h₁**, then the gas valve **6** closes and the gas valve **10** opens. In the bottom part, connecting both bioreactor arms, in the sedimentation zone, separated is the fraction that requires longer digestion time, which digestion proceeds then for the extended time in the integrated chamber **8,** designed for the slow-decomposing sedimenting fraction, while the mineral and non-biodegradable constituents are periodically, every 30 days, removed through the valve **9** to the receiver **18.** The receiver **18** is in a form of a porous rock, wherein the digestate saturated with the carbon dioxide and also non-biodegradable wastes are collected under pressure.

In the zone of decreasing hydrostatic pressure, located in the second arm **5,** the carbon dioxide, produced by the anaerobic digestion and containing traces of methane, is transferred into the biogas tank **11,** through the computer-controlled gas valve **10,** while the digestate is removed from the bioreactor through the digestate valve **12,** into the digestate tank **13.**

### Embodiment 2

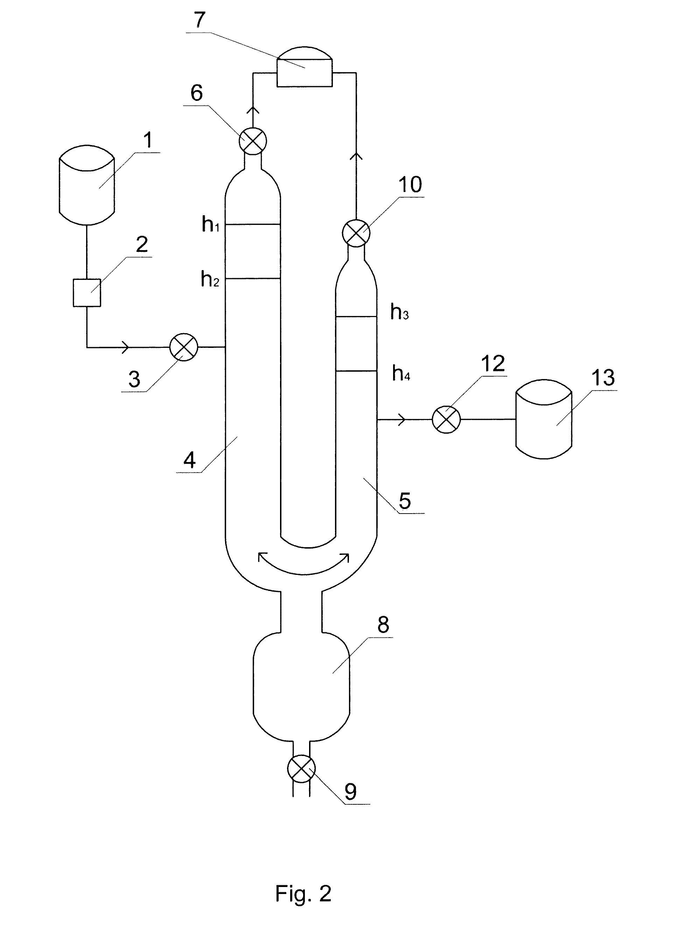

The system (visualised in Fig. 2), as just as in the embodiment 1, comprises a substrate tank **1,** from which through a metering pump **2** and a non-return valve **3,** the substrate is delivered to the first arm **4** of the bioreactor shaped in form of a letter "U", whereas the second arm **5** thereof, of a smaller diameter and height, constitutes communicating vessels with the first arm. However, the difference consists in that the produced biogas is removed from the methanogenesis zone **4** through the computer-controlled gas valve 6 into the gas tank 7, into which through the gas valve **10** is also transferred the carbon dioxide containing traces of methane, and coming from the zone of decreasing hydrostatic pressure, located in the second arm **5.** The mineral and non-biodegradable constituents are removed from the chamber **8,** designed for the slow-decomposing sedimenting fraction, through the valve **9** into the receiver, while the digestate saturated with the carbon dioxide is transferred from the zone of decreasing hydrostatic pressure, located in the second arm 5, through the valve 12 into the digestate tank **13.**

### Embodiment 3

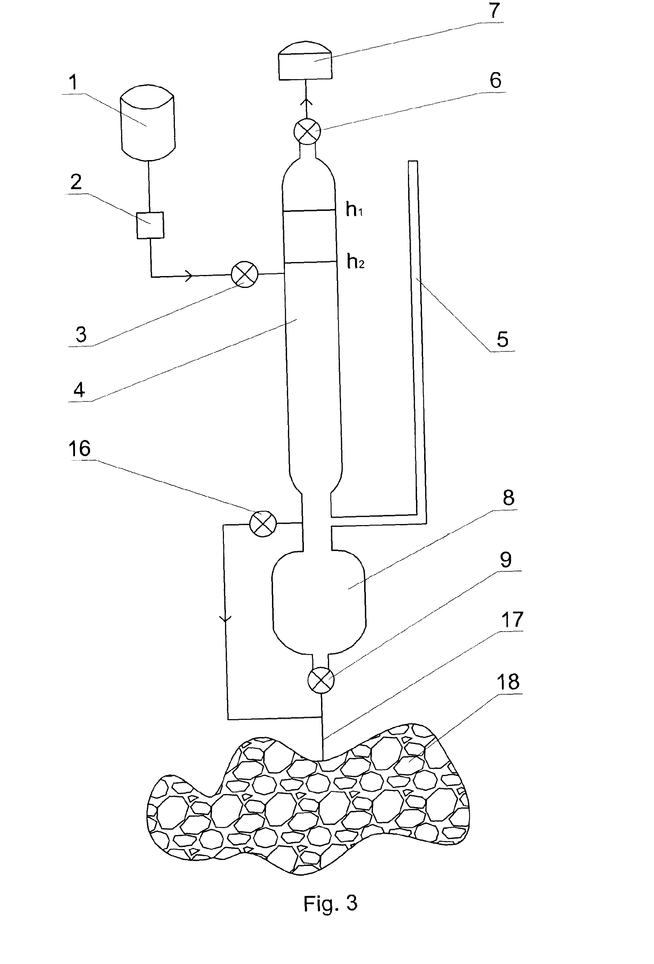

The system (visualised in Fig. 3), comprises a substrate tank **1,** from which through a metering pump **2** and a non-return valve **3,** the substrate is delivered to the bioreactor **4** in form of a long pipe. The pipeline that delivers the substrate is located in the upper part of the bioreactor, in the zone of organic matter anaerobic digestion. The produced separated methane is transferred from the bioreactor through the computer-controlled gas valve **6,** into the biogas tank 7. The separation zone in the bottom part of the bioreactor **4** is integrated with the chamber **8** for the slow-decomposing sedimenting fraction, wherefrom the mineral and non-biodegradable constituents are removed through the computer-controlled gas valve **9** and by the waste discharge pipeline **17,** which is also supplied through the valve **16** with the digestate that has been separated in the separation zone. The wastes saturated with carbon dioxide are collected under pressure in the receiver **18,** which is a rock workings.

The sedimentation zone, located in the lower part of the bioreactor **4,** is connected to the zone of decreasing hydrostatic pressure, located in the ascending vessel **5**.

### Embodiment 4

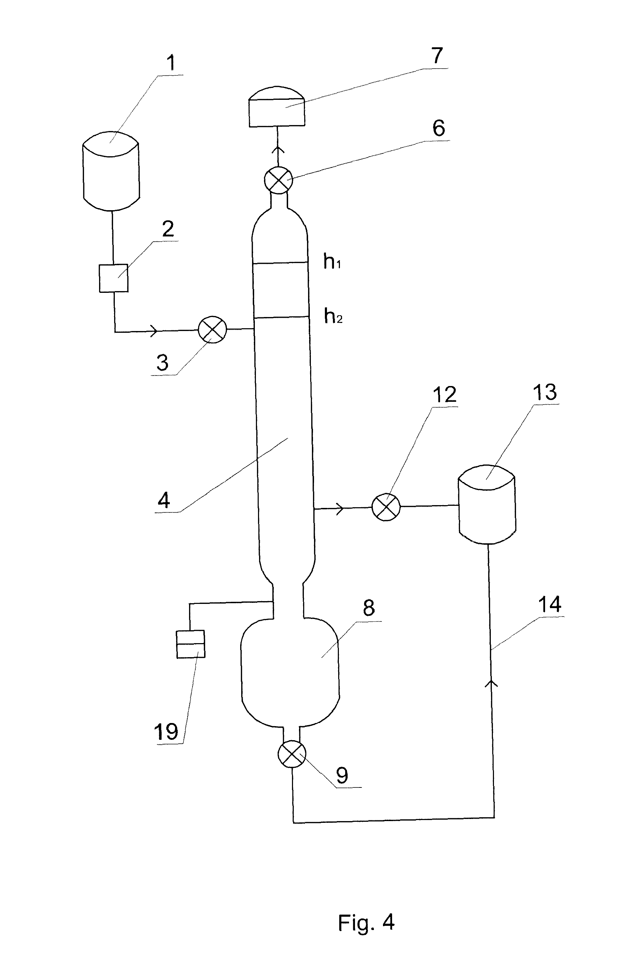

The system (visualised in Fig. 4), comprises a substrate tank **1,** from which through a metering pump **2** and a non-return valve **3,** the substrate is delivered to the bioreactor **4** in form of a long pipe. The pipeline that delivers the substrate is located in the upper part of the bioreactor, in the zone of organic matter anaerobic digestion. The produced separated methane is transferred from the bioreactor through the computer-controlled gas valve **6,** into the biogas tank 7. The separation zone in the bottom part of the bioreactor **4** is integrated with the chamber **8** for the slow-decomposing sedimenting fraction, wherefrom the mineral and non-biodegradable constituents are removed through the computer-controlled valve **9** and by the digestate discharge pipeline **14,** supplied also through the valve **12** with the digestate that has been separated in the separation zone.

The sedimentation zone, located in the lower part of the bioreactor **4,** is connected to the ascending vessel **19.**

## Claims

1. A method of methane production and disposal of carbon dioxide, comprising an organic matter methane digestion, **characterized in that** the microbiological decomposition of organic matter occurs under increasing pressure as the process progresses, in a tubular bioreactor, divided into spontaneously separating zones, where in the first zone (4) proceeds the anaerobic digestion of organic matter under increasing hydrostatic pressure as the process progresses, and methane produced as a result of the anaerobic digestion is periodically removed into the methane tank (7), and wherein the sedimentation and separation of the fraction requiring longer biodegradation time takes place in the lower bioreactor zone and the said fraction is subject to digestion for an extended period of time in the zone (8) - the lowest located zone in the bioreactor, moreover, the mineral and non-biodegradable constituents are periodically removed in the last bioreactor zone (5), wherein the hydrostatic pressure decreases as the process progresses, and the evolving gaseous carbon dioxide is removed into a gas tank, whereas the digestate is separated.

2. A method according to the claim 1, **characterized in that** the substrate feed to the bioreactor is controlled, and the digestate removal is conditioned by different conversion rates of the substrates.

3. A method according to the claim 1, **characterized in that** the reagents are mixed with microorganisms during microbiological decomposition, owing to pulsating reciprocating flow of a liquid fraction between the bioreactor zones, caused by pressure changes in the said zones.

4. A method according to the claim 1, **characterized in that** the produced carbon dioxide together with the digestate are removed into a digestate tank where the pressure is significantly lower.

5. A method according to the claim 1, **characterized in that** the carbon dioxide removed from the system together with the digestate and non-biodegradable waste is stored underground under pressure, in a rock mass void or in porous rock or at the bottom of a considerably deep water reservoir.

6. A system for methane production and disposal of carbon dioxide in the process of organic matter methane digestion, provided with a bioreactor, **characterized in that** the said bioreactor in form of a long pipe or a system of pipes, featuring spontaneously separating zones, is connected in the first zone (4) of the anaerobic digestion of organic matter to a substrate tank (1) through a substrate metering pump (2) and a substrate valve (3), and the upper part of the said bioreactor is connected to a biogas tank (7), through a gas valve (6), through which the obtained methane, separated from the liquid phase, is removed, whereas the lower part of the said bioreactor, including a sedimentation zone, ends with a digestion chamber (8) of the slow-decomposing fraction, while in the further following part (5) of the said bioreactor, with the zone of decreasing hydrostatic pressure, CO₂ is released and the digestate separated, and where in, furthermore, the sedimentation zone, integrated with the said digestion chamber (8) of the slow-decomposing fraction, is connected to a set of pipelines and valves to control the removal of mineral and non-biodegradable constituents and CO₂ from the said bioreactor.

7. A system according to the claim 6, **characterized in that** the waste valves (9, 12, 15 or 16), the said system is provided with, afford possibility to periodically remove the digestate, mineral and non-biodegradable constituents.

8. A system according to the claim 6, **characterized in that** a bioreactor is in form of a "U" -pipe, and the first arm (4) of the said "U" -pipe, including a zone of the organic matter anaerobic decomposition, is connected at the top to a methane tank (7), while the second arm (5), including a decreasing hydrostatic pressure zone, is connected to a tank (11) designed for carbon dioxide containing traces of methane, and is furthermore provided with an outflow pipe, the said pipe provided with a valve (12), for removing the digestate saturated with CO₂ to a receiver tank (13).

9. A system according to the claim 6, **characterized in that** the said system is provided with computer-controlled gas valves (6 and 10) and waste valves (9, 12, 15 or 16), making possible to maintain optimal pressure during processing the organic matter, and to control the reciprocating movement of a reaction mixture between the said bioreactor arms.

10. A system according to the claim 6, **characterized in that** a porous rock or an underground workings or a considerably deep water reservoir constitutes a waste receiver (18) for collecting a digestate saturated with carbon dioxide and non-biodegradable waste, removed from the said system by a waste discharge pipeline (17).

11. A system according to the claim 6, **characterized in that** the wastes from the said digestion chamber (8) of the slow-decomposing fraction, are fed into a digestate tank (13) through a valve (15).
